# FASCICULE DE BREVET EUROPEEN

(11) **EP 0 726 757 B1**
(45) Date de publication et mention de la délivrance du brevet: **03.01.2001**
(21) Numéro de dépôt: 95900176.9
(22) Date de dépôt: 28.10.1994
(51) Int. Cl.: A61K 7/42, A61K 7/02

(54) **COMPOSITION DERMATO-COSMETIQUE, NOTAMMENT POUR LA PHOTOPROTECTION ET PROCEDE DE PREPARATION**
DERMATOLOGISCHE UND/ODER KOSMETISCHE ZUSAMMENSETZUNG, INSBESONDERE ZUM LICHTSCHUTZ, UND HERSTELLUNGSVERFAHREN
DERMATOLOGICAL AND/OR COSMETIC COMPOSITION, IN PARTICULAR FOR SUN PROTECTION, AND METHOD FOR PREPARING SAME

(30) Priorité: 02.11.1993 FR 9312998
(43) Date de publication de la demande: 21.08.1996
(73) Titulaire: Pierre Fabre Dermo-Cosmetique, 92100 Boulogne (FR)
(72) Inventeur: MSIKA, Philippe, F-31000 Toulouse (FR); COUTELLE, Hervé, F-31500 Toulouse (FR)
(74) Mandataire: Warcoin, Jacques
(86) Numéro de dépôt international: FR9401261
(87) Numéro de publication internationale: WO9512381

(56) Documents cités:
- EP-A- 0 433 086
- EP-A- 0 530 085
- EP-A- 0 535 972
- EP-A- 0 559 320
- EP-A- 0 599 687
- EP-A- 0 610 926
- WO-A-94/17780

## Description

La présente invention se rapporte à de nouvelles compositions pour usage topique, en dermatologie et/ou cosmétologie.

La multiplication des principes actifs pouvant être employés en applications topiques a conduit à rechercher des formulations permettant de faire coexister au sein d'une même composition, des principes actifs présentant des propriétés physico-chimiques très éloignées.

Pour résoudre les phénomènes d'incompatibilité entre les différents composants, les formulations sous forme d'émulsion eau/huile ou huile/eau ont été largement utilisées.

Toutefois, elles nécessitent la présence d'un ou plusieurs tensioactifs, qui ont généralement une action irritante sur la peau. En outre, dans le cas où les compositions renferment un agent particulaire, des problèmes de texture et d'acceptabilité cosmétique se posent, qui interdisent de trop fortes concentrations de particules et obligent à augmenter les concentrations des autres principes actifs. Enfin, après l'application, la persistance du film formé à la surface de la peau est réduite, en particulier pour les émulsions huile/eau car l'émulsion se rompt facilement en présence d'eau ou de transpiration ; ceci est particulièrement gênant dans le cas de compositions photoprotectrices destinées à être appliquées chez des sujets pouvant se baigner et chez lesquels il importe de maintenir une protection durable et efficace ; dans ce dernier cas, les manifestations d'intolérance engendrées par la présence de nombreux filtres (par exemple oxybenzone et PABA) créent un besoin pour des compositions où la teneur en ces composants pourrait être réduite.

La demande de brevet EP-A-0 610 926 fait partie de l'état de la technique visé à l'article 54 (3) CBE. Ce document décrit des compositions destinées à lutter contre l'action urticante de certains types de méduses. Ces compositions se présentent sous la forme d'hydrodispersions contenant des micropigments minéraux et éventuellement des filtres anti-UV, les micropigments minéraux étant de préférence dans les phases lipidiques des hydrodispersions.

EP-A-0 530 085 décrit une composition cosmétique pour le maquillage de la peau contenant au moins une cire de silicone dispersée dans une phase aqueuse contenant deux types de polymère(s) acrylique(s) hydrosoluble(s) tels que l'on obtient une dispersion stable de cire(s) de silicone, contenant plus de 10 % en poids de cire de silicone et des dispersions stables contenant jusqu'à 60% en poids de cire(s) de silicone. Les compositions selon EP-A-0 530 085 peuvent contenir des charges telles que des pigments minéraux mélangées à la phase grasse.

Ces buts, et d'autres qui apparaîtront dans la suite de la description, peuvent être atteints avec une composition dermato-cosmétique caractérisée en ce qu'elle consiste en une dispersion stable de microsphères qui comprend :
- une phase huileuse, contenant au moins un oxyde métallique,
- un gel aqueux, constituant la phase continue,
- un agent isolant lipophile.

Dans les compositions selon l'invention, l'agent isolant lipophile est un agent de surface dont le HLB est inférieur ou égal à 3.

Les compositions selon l'invention sont des microdispersions. A la différence des émulsions de l'art antérieur, elles ne contiennent aucun constituant jouant sur la tension interfaciale classé dans les émulsionnants eau/huile ou huile/eau.

Il n'y a donc pas d'abaissement de la tension interfaciale avec formation de structure intermédiaire lamellaire, mais au contraire les agents de HLB ≤ 3 augmentent ce paramètre et dissocient la phase huileuse de la phase aqueuse.

En outre, dans le cas particulier des produits photoprotecteurs, la phase continue huileuse, était considérée comme indispensable pour une formulation satisfaisante, en particulier pour des photoprotecteurs lipophiles.

L'incorporation de la phase huileuse, contenant les oxydes métalliques dans la phase gel, met en évidence une incompatibilité notoire entre la phase gel et les oxydes métalliques. Il se forme un gel cassant, exsudant, rendant impossible la préparation de la microdispersion.

Cette incompatibilité a été surmontée de manière inattendue par les compositions de la présente invention.

Le gel aqueux comprend de préférence un polymère d'acrylate et/ou alkyle acrylate. De manière avantageuse, on utilise des polymères ramifiés d'acrylate/C₁₀-C₃₀ alkylacrylates du type Pemulen ® commercialisés par BF Goodrich, en particulier les Pemulen ® TR1 et TR2. Le polymère acrylate est de préférence présent à raison de 0,01 à 2% en poids de la composition finie.

Un tel gel est constitué d'un réseau, dans lequel sont dispersées les microsphères huileuses en présence de l'agent de surface de HLB ≤ 3.

Parmi les agents isolants liphophiles convenant pour les compositions selon l'invention, on peut citer : trioléate de sorbitan, tristéarate de sorbitan, diglycérol tétrastéarate, ester de polyglycérol et d'acides gras, décaglycérol décastéarate, glycérol mono et dioléate et propylène glycol, glycérol mono et dioléate, sorbide dioléate, POE-1 nonylphénol, perfluor polyméthyl- isopropyl ether.

L'agent isolant lipophile est de préférence présent à une concentration comprise entre 0,1 et 5% en poids de la composition finie.

D'autres éléments peuvent être incorporés dans la phase aqueuse, afin notamment d'augmenter sa stabilité, de modifier la viscosité, la rhéologie et le toucher de ces gels, et notamment tout élément gélifiant d'origine naturelle ou de synthèse type Alginate, carraghénate, gomme Xanthane, Carbomer : Carbopol® 934, 980, 940... ETD 2001, dérivé de cellulose (Méthocel®...)

D'autres molécules hydrophiles peuvent être incluses pour donner un caractère hydratant, émollient et aussi des filtres solaires, hydrosolubles (Eusolex® 232...).

De manière facultative, le gel aqueux des compositions selon l'invention contient un polymère carboxyvinylique, à une concentration comprise entre 0 et 5%.

Les microsphères de la phase huileuse contiennent des particules dont le diamètre est de préférence inférieur ou égal à 20 µm. Les compositions selon la présente invention sont particulièrement adaptées à la formulation de particules de diamètre compris entre 10 et 200 nm, contenues dans les microsphères de la phase huileuse.

Selon l'un des aspects de l'invention, les particules sont choisies parmi les écrans minéraux ultrafins enrobés ou non, sous forme de poudre, d'empàtage huileux, de dispersion prête à l'emploi, comme par exemple :
. Tio₂ ultrafin (de 10 à 150 nm)
   - P 25 et T 805 (Degussa)
   - MT100 T (Unipex)
   - TiOveil (Tioxide)
. ZnO ultrafin (de 10 à 200 nm)
   - Z Cote (Unipex)
   - Zinc Neutral (Harmann et Rheimer)
   - Spectraveil® (Tioxide)
. Autres oxydes métalliques (fer, cerium, aluminium, zirconium etc.)

Lesdites particules peuvent également être choisies parmi les mélanges de pigments de plus forte granulométrie comme l'oxyde de fer, micatitane, Tio₂, ZnO, Talc, de taille particulaire inférieure à 20 µm. Ils peuvent, de manière facultative, être additionnés de sulfate de baryum ou nitrure de Bore.

De préférence, les oxydes métalliques nanoparticulaires ou microparticulaires, sont enrobés par des composés lipophiles afin d'isoler électriquement les minéraux et d'optimiser leur stabilité dans la formulation.

L'enrobage peut se réaliser par tous composés isolants (lécithines, alumine, acide stéarique, acide aminé, ester, silicone...) antiradicalaires ou anti-oxydants, voire par certains filtres organiques liposolubles.

Le ou les oxydes métalliques sont de préférence présents à raison de 0,1 à 25% en poids de la composition finie.

La phase huileuse peut contenir en outre différents éléments entrant habituellement dans la constitution des phases huileuses des produits cosmétiques, esters, silicone volatil ou rémanent, principe actif huileux, des dérivés monoparticulaires de 10 à 40 nm, d'origine biologique, type Eumélanine purifiée et nanomisée (Sépia-Mélanink-Melio-California), des conservateurs, des parfums, huiles végétales, huiles minérales, antioxydants, etc.

Selon un aspect particulièrement avantageux de l'invention, les compositions sont des compositions photoprotectrices consistant en une microdispersion d'une phase huileuse comportant des microparticules, dans un gel aqueux en présence d'un agent de surface de HLB inférieur ou égal à 3, et qui contient en outre au moins un filtre organique.

Les filtres organiques liposolubles sont présents dans la phase huileuse et peuvent notamment être choisis parmi les composés suivants, aux concentrations maximales indiquées :

| **SUBSTANCES** | **CONCENTRATION MAXIMALE AUTORISEE** |
|---|---|
| - Acide 4 aminobenzoïque | 15% |
| - Sulfate de méthyle de N.N. N-triméthyl (oxo-2-bornylidène-3) méthyl-4 anilinium | 6% |
| - Homosalate (DCI) | 15% |
| - Oxybenzone (DCI) | 10% |
| - Acide-3 Imidazol-4 yl acrylique et son ester éthylique | 2% (exprimé en acide) |
| - 3-3'-(1,4 phénylénediméthylidine) bis (7,7-diméthyl-2-oxobicyclo-2,2,1) heptane-1-méthanesulfonique acide) et ses sels | 10% (exprimé en acide) |
| - 4-N Dipropoxy aminobenzoate d'éthyl (mélange d'isomères) | 5% |
| - 4-polyéthoxy aminobenzoate d'éthyl | 10% |
| - 1-(4-aminobenzoate) de glycérol | 5% |
| - 4-(diméthylamino)-benzoate d'éthyl-2 hexyle | 8% |
| - Salicylate d'éthyl-2 hexyle | 5% |
| - 4-méthoxycinnamate d'isopentyl (mélange d'isomères) | 10% |
| - 4-méthony cinnamate d'éthyl-2 hexyle | 10% |
| - 2-hydroxy 4-méthoxy 4-méthylbenzophénone (mexenone (DCI) ) | 4% |
| - Dioxybenzone | 3% |
| - Menthyl anthranilate | 5% |
| - Amyl diméthyl PABA | 5% |
| - Ethoxy éthyl p-méthoxycinnamate | 3% |
| - Diéthanolamine p-méthoxycinnamate | 10% |
| - Digalloyl trioléate | 5% |
| - Octocrylène | 10% |
| - TEA salicylate | 12% |
| - Benzophénone-1 | 3% |
| - Benzophénone-2 | |
| - Acide alpha- (oxo 2- bornylidène-3)-toluène-4 sulfonique et ses sels | 6% (exprimé en acide) |
| - 3-(4'-méthylbenzylidène) camphre | 6% |
| - 3-benzylidène camphre | 6% |
| - 4-isopropyl-dibenzoylméthane | 5% |
| - Salicylate d'isopropyl-4 benzyle | 4% |
| - Tert-butyl-4 phényl)-1 méthoxy-4 phényl)-3 propanedione-1,3 | 5% |
| - 2, 4, 6-trianilino- (p-carbo-2' éthylhexyle-1'-oxi) 1, 2, 3 triazine) | 5% |
| - Benzophénone-5 | |
| - Benzophénone-6 | |
| - Benzophénone-9 | |
| - Isopropyle méthoxycinnamate | |

En effet, la Demanderesse a montré, de manière inattendue, qu'il existe une synergie d'activité photoprotectrice entre les filtres organiques et les filtres particulaires présents dans la phase huileuse des dispersions selon l'invention.

Cette synergie a été mise en évidence par la comparaison des résultats d'un couple filtre organique/écran minéral dans un excipient classique et dans les compositions selon l'invention. Elle permet de réduire le nombre et la quantité de filtres organiques en contact avec la peau.

L'une des hypothèses, par lesquelles on n'entend nullement limiter l'invention, est que grâce à l'individualisation des globules huileux photoprotecteurs par les composés très lipophiles, on note non seulement une compatibilité remarquable avec l'excipient, mais aussi une répartition idéale des écrans minéraux à la surface de la membrane isolante.

De plus, la protection est identique dans le temps, les cristaux dispersés ne se réagglomérant pas dans la formulation. En assurant la dispersion des oxydes métalliques sur la peau après étalement de la préparation par la présence du composé de HLB ≤ 3, on potentialise la protection solaire par une synergie très importante entre l'absorption des filtres organiques et la réflexion intense des écrans minéraux dispersés.

On peut également utiliser des filtres chimiques hydrosolubles, qui sont alors présents dans la phase aqueuse continue.

La présente invention a également pour objet un procédé de préparation d'une composition dermatologique et/ou cosmétologique, caractérisée en ce que :
a) on effectue un mélange homogène des microparticules avec les autres composants de la phase huileuse,
b) on ajoute au mélange de l'étape a) un agent isolant lipophile,
c) on prépare un gel comprenant un polymère acrylique dans une phase aqueuse,
d) on effectue la dispersion sous agitation du mélange obtenu à l'issue de l'étape b) dans la phase aqueuse obtenue à l'issue de l'étape c),
e) on neutralise la dispersion obtenue par une base, afin d'obtenir une microdispersion présentant un pH compris entre 6 et 8.

La formulation des compositions selon l'invention est donc effectuée par exemple de la façon suivante :
1) Préparation de la phase aqueuse 1% à 99%
   . Pemulen
   . Carbomer
   . Methocel HC
   . Ingrédients aqueux
   Les composants sont dispersés dans l'eau et homogénéisés.
2) Préparation de la phase huileuse 1% à 80%
   . Oxydes métalliques ultrafins ou pigmentaires enrobés
   . Filtres organiques UVB-UVA
   . Ingrédient huileux
   Mélanger de façon homogène à froid ou à chaud.
3) Isolant lipophile 0,1% à 5%
   Ajouter l'agent de surface à la phase huileuse.
4) Réaliser la dispersion sous très forte agitation de la phase huileuse dans la phase aqueuse (l'inverse étant aussi possible).
5) Neutralisation de la phase externe par une base type TEA ou AMP.

Les exemples qui suivent sont destinés à illustrer l'invention sans aucunement en limiter la portée.

Dans ces exemples on se référera aux figures suivantes :
- **Figure 1 :**: Comparaison des facteurs de protection solaire (FPS) obtenue avec 1% de Tio₂ et des concentrations variables de cinnamate, dans des compositions selon l'art antérieur et dans des compositions selon l'invention.
- **Figure 2 :**: Comparaison des IP anti UVB avec 8% de cinnamate couplé à des concentrations croissantes de Tio₂ ultrafin dans des compositions selon l'art antérieur et dans des compositions selon l'invention.

### Exemple 1 : Crème bébé à l'oxyde de zinc sans émulsionnant

- Oxyde de zinc 0,5% à 20%
- Huile de poisson 0,1% à 10%
- Huile minérale 0,1% à 20%
- Polyglycéryl 2 tétrastéarate 0,1% à 5%
   . extrait de calendula 0 à 10%
   . acrylates
      (polymère réticulé d'alkylacrylates en C10-C30) 0,01% à 1%
- Carbomère 0 à 1%
- Gomme xanthane 0 à 1%
- Eau
   Neutralisation pH ≃ 6,5 à 7

### Exemple 2 : Fond de teint sans émulsionnant

- Trioléate de Sorbitan 0,1% à 2%
- Nylon 0 à 5%
- Lauroyl Lysine 0 à 10%
- Talc 1% à 10%
- Dioxyde de titane 1% à 10%
- C.I. 77492 0,01% à 2%
- C.I. 77491 0,01% à 2%
- C.I. 77499 0,01% à 1%
- Esters 1% à 25%
- Conservateurs qsp
   Pemulen® TR1 0,01% à 1%
   Carbopol® 940 0 à 1%
   Veegum® 0 à 1%

   Eau qsp
   TEA pH ≃ 6,5

### Exemple 3 : Crème solaire

- Dibenzoylméthane 0,1% à 5%
- Octyl méthoxycinnamate 0,1% à 10%
- Dioxyde de titane 0,1% à 25%
- Paraffine et dioxyde de titane et oxyde de fer et méthicone 0 à 15%
- Alkylbenzoate en C12 - C 15 0,5% à 15%
- Octyldodécyl néopentanoate 0,5% à 15%
- Monoï 0 à 5%
- Alcool cétylique 0 à 1%
- Cyclométhicone et diméthiconol 0 à 10%
- Trioléate de sorbitan 0,1% à 5%
- Acétate de tocophérol 0 à 1%
- Eau ou eau de la source thermale d'Avène qsp
- Glycérine 0 à 10%
- Polymère réticulé acrylates / alkylacrylates en C 10 - C 30 0,01% à 1%
- Carbomère 0 à 1%
- Hydroxypropylméthylcellulose 0 à 1%
- Conservateurs qsp
- EDTA disodique 0 à 0,3%
- Parfum
- Triéthanolamine qsp pH 6 à 8
- Capteur de radicaux libres 0 à 2% (flavonoïdes, extrait d'hibiscus, carotène)

### Exemple 4 : Crème solaire sans filtre chimique

- Talc 0 à 10%
- Dioxyde de titane 0,5% à 25%
- Oxyde de zinc 0,5% à 10%
- Sulfate de baryum 0 à 5%
- Polyglycéryl-10 décastéarate 0 à 5%
- Huile végétale 1% à 20%
- Huile minérale 1% à 20%
- Polymère réticulé acrylates / alkylacrylates en C 10 - C 30 0,01% à 1%
- Carbomère 0 à 1%
- Gomme xanthane 0 à 1%
- Eau qsp
- Conservateurs qsp
- TEA ou AMP pH 6 à 8

### Exemple 5 : Spray solaire

- Cinnamate 0 à 10%
- Suspension de dioxyde de titane 0,5% à 20%
- Alkylbenzoate en C 12 - C 15 0 à 10%
- Octyldodécyl néopentanoate 0 à 10%
- Trioléate de sorbitan 0,1% à 5%
- Huile minérale 1% à 10%
- Conservateurs qsp
- Eau qsp
- Pemulen TR2 0,01% à 1%
- Gomme xanthane 0 à 1%
- TEA qsp pH 6 à 8

### Exemple 6 : Crème solaire spectre large

- Cinnamate 0,5% à 10%
- Dispersion de dioxyde de titane dans l'huile 0,5% à 25%
- POE - 1 nonyl phénol 0,1% à 5%
- Huile minérale 1% à 20%
- Polymère réticulé acrylates / alkylacrylates en C 10 - C 30 0,01% à 1%
- Carbomère 0 à 1%
- Hydroxypropylméthylcellulose 0 à 1%
- AMP PH = 6,5 à 7
- EAU qsp

### Exemple 7 : Mise en évidence de la synergie de photoprotection

L'ensemble des tests décrits ci-dessous, a été réalisé aux Laboratoires du Docteur CESARINI : Photobiologiste INSERM LR.T.P.H. 25 rue Manin - 75019 PARIS-

Le protocole suivi pour les études de la protection UVB sur volontaires sains, est celui de la recommandation FDA (Sunscreen products for over-the-counter Fed. Reg., 3 : 28269 (1978).

Les résultats sont enregistrés et déposés au LR.T.P.H.

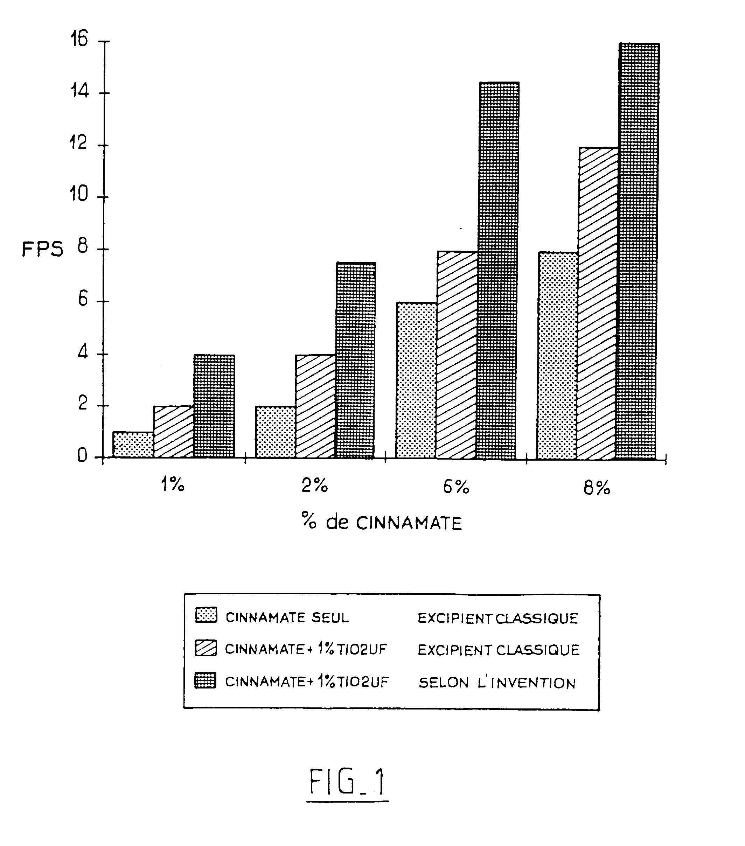

Le tableau et la figure 1 ci-après relatent la différence de synergie d'un couple (cinnamate + Ti02 ultrafin) dans un excipient classique (type eau/silicone) et dans l'excipient relaté dans l'invention.

Pour base de comparaison, nous avons identifié les résultats de protection du Cinnamate seul dans l'excipient classique eau/silicone.

**TABLEAU 1 ;**

| **SYNERGIE DE PROTECTION SUIVANT L'EXCIPIENT POUR 1% DE Tio**_{**2**} **ULTRAFIN ASSOCIE A DES QUANTITES VARIABLES DE CINNAMATE**. | | | | |
|---|---|---|---|---|
| % de Cinnamate photoprotecteur | 1% | 2% | 6% | 8% |
| | | | | |
| | Indice de protection UVB | | | |
| | | | | |
| CINNAMATE | 1 | 2 | 6 | 8 |
| | | | | |
| CINNAMATE + Tio₂ classique | 2 | 4 | 8 | 12 |
| | | | | |
| CINNAMATE + Tio₂ + excipient selon l'invention | 4 | 8 | 14 | 16 |

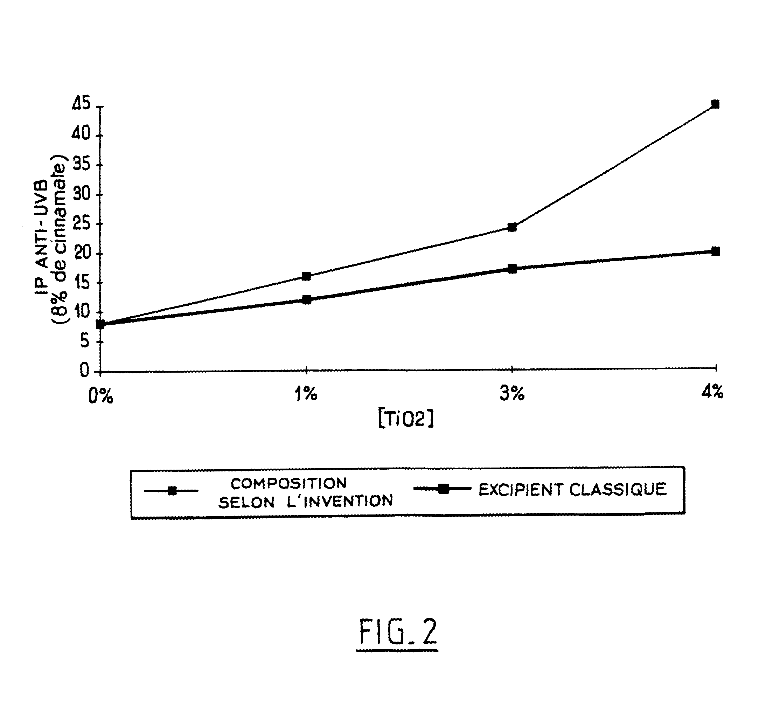

Une synergie d'activité est également observée pour des compositions contenant une concentration de 8% en poids de cinnamate et des concentrations croissantes de TiO₂. Ces résultats comparatifs sont représentés sur la figure 2 en annexe.

### Exemple 8 : Preuve de photostabilité et de sécurité de la formulation

Quatre formules préparées selon l'invention, testées en Laboratoire pour avoir des I.P. variant de 4 à 25, ont été éprouvées par un calcul de l'indice de protection en conditions naturelles d'ensoleillement, de nage et de sueur sous le contrôle du Docteur CESARINI (LRTPH ; INSERM).
**Crème n° 1 :**
   . Cinnamate 1%
   . Tio₂ 1%
   dans l'excipient se référant à l'invention.
**Crème n° 2 :**
   . Cinnamate 2%
   . Tio₂ 1%
**Crème n° 3 :**
   . Cinnamate 6%
   . Tio₂ 1%
**Crème n° 4 :**
   . Cinnamate 8%
   . Tio₂ 3%

Le protocole est adapté des recommandations de la FDA et de la norme australienne de rémanence à l'eau.

Après application sur treize sujets volontaires sains, des quatre crèmes (à des doses de 1 à 3 mg/cm², selon les indices), les volontaires subissent deux épreuves de baignade en eau de mer de 20 minutes alternées par une période de séchage à l'air libre de 10 minutes.

Après une exposition solaire de 1 à 5 heures : proportionnelle à la protection théorique à la dose de produit appliquée et à la sensibilité individuelle des sujets, on calcule l'I.P. des produits et la rémanence.

Les résultats sont les suivants :

**TABLEAU 2**

| | IP Labo | IP extérieur | Réman. labo | Réman. piscine |
|---|---|---|---|---|
| Crème n° 1 | 5 | 4 | 85 | 85 |
| Crème n° 2 | 8 | 7 | 79 | 100 |
| Crème n° 3 | 15 | 17 | 94 | 86 |
| Crème n° 4 | 21 | 22 | 88 | 77 |

On remarque, qu'au bout d'une journée de plage d'ensoleillement, de baignade et de sueur dans des conditions optimales, les protections solaires sont tout aussi efficaces qu'en Laboratoire.

## Revendications

1. Composition dermato-cosmétique caractérisée en ce qu'elle consiste en une dispersion stable de microsphères qui comprend :
- une phase huileuse, contenant au moins un oxyde métallique,
- un gel aqueux, constituant la phase continue,
- un agent isolant lipophile,
l'agent isolant lipophile étant un agent de surface dont le HLB est inférieur ou égal à 3.

2. Composition selon la revendication 1, caractérisée en ce que l'agent isolant lipophile est choisi dans le groupe comprenant : trioléate de sorbitan, tristéarate de sorbitan, diglycérol tétrastéarate, ester de polyglycérol et d'acides gras, décaglycérol décastéarate, glycérol mono et dioléate et propylène glycol, glycérol mono et dioléate, sorbide dioléate, POE-1 nonylphénol, perfluor polyméthylisopropyl ether.

3. Composition selon l'une des revendications 1 et 2, caractérisée en ce que les microsphères de la phase huileuse comprennent des particules de diamètre inférieur ou égal à 20 µm.

4. Composition selon l'une des revendications 1 à 3, caractérisée en ce que les microsphères de la phase huileuse contiennent des particules de diamètre compris entre 10 et 200 nm.

5. Composition selon l'une des revendications 3 et 4, caractérisée en ce que l'oxyde métallique est choisi dans le groupe comprenant le dioxyde de titane et les oxydes de zinc, de fer, d'aluminium, de zirconium et de cerium.

6. Composition selon l'une des revendications 1 à 5, caractérisée en ce que le gel aqueux comprend un polymère d'acrylate et/ou alkyle acrylate.

7. Composition selon l'une des revendications 1 à 6, caractérisée en ce que le gel aqueux contient un polymère carboxyvinylique.

8. Composition selon l'une des revendications 1 à 7, caractérisée en ce qu'il s'agit d'une composition photoprotectrice qui comporte en outre au moins un filtre organique dans la phase huileuse.

9. Composition selon la revendication 8, caractérisée en ce que le filtre organique est choisi parmi les filtres UVB et/ou UVA liposolubles.

10. Composition selon l'une des revendications 1 à 9, caractérisée en ce que l'oxyde métallique est présent à raison de 0,1 à 25% en poids de la composition finie.

11. Composition selon l'une des revendications 1 à 10, caractérisée en ce que l'isolant lipophile est présent à raison de 0,1 à 5% en poids de la composition finie.

12. Composition selon l'une des revendications 6 à 11, caractérisée en ce que le polymère acrylate est présent à raison de 0,01 à 2% en poids de la composition finie.

13. Procédé de préparation d'une composition dermato-cosmétique selon l'une des revendications 1 à 12, caractérisé en ce que :
a) on effectue un mélange homogène des particules d'oxyde métallique avec les autres composants de la phase huileuse,
b) on ajoute au mélange de l'étape a) un agent isolant lipophile,
c) on prépare un gel comprenant un polymère acrylique dans une phase aqueuse,
d) on effectue la dispersion sous agitation du mélange obtenu à l'issue de l'étape b) dans la phase aqueuse obtenue à l'issue de l'étape c),
e) on neutralise la dispersion obtenue par une base, afin d'obtenir une microdispersion présentant un pH compris entre 6 et 8.

14. Composition selon les revendications 1 à 10, caractérisée en ce que la phase huileuse peut contenir un dérivé monoparticulaire d'origine biologique de type Eumélanine.

## Patentansprüche

1. Dermato-kosmetische Zusammensetzung, dadurch charakterisiert, daß sie aus einer stabilen Dispersion von Mikrokügelchen besteht, welche umfaßt:
- eine ölige Phase, enthaltend mindestens ein Metalloxid,
- ein wäßriges Gel, das die kontinuierliche Phase darstellt,
- ein lipophiles isolierendes Mittel, welches ein oberflächenaktiver Stoff ist, dessen HLB kleiner oder gleich drei ist.

2. Zusammensetzung nach Anspruch 1, dadurch charakterisiert, daß das isolierende lipophile Mittel ausgewählt wird aus der Gruppe, welche umfaßt: Sorbitantrioleat, Sorbitantristearat, Diglycerintetrastearat, Ester von Polyglycerin und Fettsäuren, Decaglycerindecastearat, Glycerinmono- und dioleat und Propylenglykol, Glycerinmono-und dioleat, Sorbitdioleat, POE-1-nonylphenol, Perfluorpolymethylisopropylether.

3. Zusammensetzung nach einem der Ansprüche 1 und 2, dadurch charakterisiert, daß die Mikrokügelchen der öligen Phase Partikel mit einem Durchmesser von weniger oder gleich 20 µm umfassen.

4. Zusammensetzung nach einem der Ansprüche 1 bis 3, dadurch charakterisiert, daß die Mikrokügelchen der öligen Phase Partikel mit einem Durchmesser zwischen 10 und 200 nm enthalten.

5. Zusammensetzung nach einem der Ansprüche 3 und 4, dadurch charakterisiert, daß das Metalloxid ausgewählt wird aus der Gruppe, die Titandioxid und die Oxide von Zink, Eisen, Aluminium, Zirkonium und Cer umfaßt.

6. Zusammensetzung nach einem der Ansprüche 1 bis 5, dadurch charakterisiert, daß das wäßrige Gel ein Acrylat- und/oder Alkylacrylat-Polymer umfaßt.

7. Zusammensetzung nach einem der Ansprüche 1 bis 6, dadurch charakterisiert, daß das wäßrige Gel ein Carboxyvinyl-Polymer enthält.

8. Zusammensetzung nach einem der Ansprüche 1 bis 7, dadurch charakterisiert, daß es sich um eine Lichtschutz-Zusammensetzung handelt, die darüber hinaus mindestens einen organischen Filter in der öligen Phase umfaßt.

9. Zusammensetzung nach Anspruch 8, dadurch charakterisiert, daß der organische Filter ausgewählt wird aus den fettlöslichen UVB- und/oder UVA-Filtern.

10. Zusammensetzung nach einem der Ansprüche 1 bis 9, dadurch charakterisiert, daß das Metalloxid in einem Verhältnis von 0,1 bis 25 Gew.-% der End-Zusammensetzung vorhanden ist.

11. Zusammensetzung nach einem der Ansprüche 1 bis 10, dadurch charakterisiert, daß das lipophile isolierende Mittel in einem Verhältnis von 0,1 bis 5 Gew.-% der End-Zusammensetzung vorhanden ist.

12. Zusammensetzung nach einem der Ansprüche 6 bis 11, dadurch charakterisiert, daß das Acrylatpolymer in einem Verhältnis von 0,01 bis 2 Gew.-% der End-Zusammensetzung vorhanden ist.

13. Verfahren zur Herstellung einer dermato-kosmetischen Zusammensetzung nach einem der Ansprüche 1 bis 12, dadurch charakterisiert, daß
a) man eine homogene Mischung der Metalloxidpartikel mit den weiteren Bestandteilen der öligen Phase herstellt,
b) man zur Mischung des Schritts a) ein isolierendes lipophiles Mittel zugibt,
c) man ein Gel herstellt, welches ein Acrylpolymer in einer wäßrigen Phase umfaßt,
d) man unter Rühren die am Ende von Schritt b) erhaltene Mischung in der am Ende von Schritt c) erhaltenen wäßrigen Phase dispergiert,
e) man die erhaltene Dispersion mit einer Base neutralisiert, um eine Mikrodispersion zu erhalten, die einen pH zwischen 6 und 8 zeigt.

14. Zusammensetzung nach den Ansprüchen 1 bis 10, dadurch charakterisiert, daß die ölige Phase ein monopartikuläres Derivat biologischen Ursprungs vom Eumelanin-Typ enthalten kann.

## Claims

1. Dermatocosmetic composition, characterized in that it consists of a stable dispersion of microspheres which comprises:
- an oily phase, containing at least one metal oxide,
- an aqueous gel, constituting the continuous phase,
- a lipophilic insulating agent,
the lipophilic insulating agent being a surface agent whose HLB value is less than or equal to 3.

2. Composition according to Claim 1, characterized in that the lipophilic insulating agent is chosen from the group comprising: sorbitan trioleate, sorbitan tristearate, diglyceryl tetrastearate, ester of polyglycerol and of fatty acids, decaglyceryl decastearate, glyceryl mono- and dioleate and propylene glycol, glyceryl mono- and dioleate, sorbide dioleate, POE-1 nonylphenol, and perfluoropolymethylisopropyl ether.

3. Composition according to either of Claims 1 and 2, characterized in that the microspheres in the oily phase comprise particles less than or equal to 20 µm in diameter.

4. Composition according to one of Claims 1 to 3, characterized in that the microspheres in the oily phase contain particles between 10 and 200 nm in diameter.

5. Composition according to either of Claims 3 and 4, characterized in that the metal oxide is chosen from the group comprising titanium dioxide and zinc oxides, iron oxides,aluminiumoxydes, zirconium oxydes and cerium oxides.

6. Composition according to one of Claims 1 to 5, characterized in that the aqueous gel comprises an acrylate and/or alkyl acrylate polymer.

7. Composition according to one of Claims 1 to 6, characterized in that the aqueous gel contains a carboxyvinyl polymer.

8. Composition according to one of Claims 1 to 7, characterized in that it is a photoprotective composition which also comprises at least one organic screening agent in the oily phase.

9. Composition according to Claim 8, characterized in that the organic screening agent is chosen from liposoluble UVB and/or UVA screening agents.

10. Composition according to one of Claims 1 to 9, characterized in that the metal oxide is present in a proportion of from 0.1% to 25% by weight of the finished composition.

11. Composition according to any one of Claims 1 to 10, characterized in that the lipophilic insulating agent is present in a proportion of from 0.1% to 5% by weight of the finished composition.

12. Composition according to one of Claims 6 to 11, characterized in that the acrylate polymer is present in a proportion of from 0.01% to 2% by weight of the finished composition.

13. Process for preparing a dermatocosmetic composition according to one of Claims 1 to 12, characterized in that:
a) the metal oxide particles are homogeneously mixed with the other components of the oily phase,
b) a lipophilic insulating agent is added to the mixture from step a),
c) a gel is prepared comprising an acrylic polymer in an aqueous phase,
d) the mixture obtained from step b) is dispersed, with stirring, in the aqueous phase obtained after step c),
e) the dispersion obtained is neutralized with a base, in order to obtain a microdispersion with a pH of between 6 and 8.

14. Composition according to Claims 1 to 10, characterized in that the oily phase can contain a monoparticulate derivative of biological origin of eumelanin type.
